# EUROPEAN PATENT SPECIFICATION

(11) **EP 2 594 258 B1**
(45) Date of publication and mention of the grant of the patent: **18.03.2020**
(21) Application number: 11189706.2
(22) Date of filing: 18.11.2011
(51) Int. Cl.: A61K 9/00, A61K 31/495, A61K 47/10, A61K 47/38

(54) **Oral gel comprising praziquantel**
Orales Gel enthaltend praziquantel
Gel oral comprenant du praziquantel

(43) Date of publication of application: 22.05.2013
(73) Proprietor: Veterinärmedizinische Universität Wien, 1210 Vienna (AT)
(72) Inventor: Dadak, Agnes, 1020 Vienna (AT); Franz, Sonja, 2403 Wildungsmauer (AT); Liebhart, Andreas, 1110 Vienna (AT)
(74) Representative: Redl, Gerda

(56) References cited:
- WO-A1-2009/062939
- US-A1- 2003 096 822
- US-A1- 2004 151 759

## Description

The invention refers to a gel for oral administration comprising at least 15% (w/w) free praziquantel, at least 30% water and at least one gelifyer, which is devoid of cyclodextrins and cyclodextrin inclusion complex.

### Background

There are unique specifications for veterinary medical care of New World Camelids (llama, alpaca, vicuna, and guanaco; NWC). Various medications are administered via the oral route, which can be challenging when medicaments initially developed to treat equines or smaller animals are used and the proper dosing regime is different. Parasite controle of a NWC herd is particularly important because of the high risk of lethality.

Dicrocoeliosis, also known as "small liver fluke" or "lancet fluke" disease, belongs to the principal and economically most important pasture helminthiases in ruminants. The parasite *Dicrocoelium dentriticum* lives in the bile ducts and gall bladder of domestic and wild ruminants such as sheep, goats, cattle, buffalos, mufflons, fallow deer, roe deer, and camels. It occasionally affects horses, dogs, rabbits, pigs and also humans. Efficient control of the parasite is therefore highly desirable. Eggs of *Dicrocoelium dentriticum* are capable of surviving on contaminated pastures up to two years. Dicrocoeliosis is common in several regions of Europe, Asia, North Africa, North and South America and the Middle East where the local conditions are favourable for the two intermediate hosts, terrestrial snails and ants.

Dicrocoeliosis is known to be a severe endoparasitosis in NWC. NWC infected with the trematode develop liver function impairment more devastating than seen in cattle or small ruminants. Dicrocoeliosis often remains clinically undetected in NWC since these animals show no signs of disease until a very late stage. Therefore, infection with *Dicrocoelium dentriticum* most often is fatal. The diagnosis then is based on adult parasites detected in the liver post mortem. In living animals, diagnosis is based on eggs detected at coprological examinations, since there is no reliable enzyme-linked immunosorbent assay available to date.

Most antiparasitic drugs are practically ineffective against the small liver fluke. Lethal intoxications have been reported following treatment of alpacas with albendazole.

Praziquantel is the drug of choice for treatment of dicrocoeliosis in NWC so far. The recommended dose rate for llamas and alpacas is 50 mg/kg body weight (BW). Adult llamas weight about 110 - 200 kg, adult alpacas up to 55 - 90 kg. There is no authorized product on the market containing a high enough praziquantel concentration to guarantee an easy, safe and complete application of the dose required.

WO2004/000034 A2 describes anthelmintic oral homogeneous pastes comprising praziquantel and a macrolide anthelmintic compound and a solvent, which dissolves the praziquantel and a macrolide anthelmintic compound, and a thickening agent. An exemplary paste comprises 7.75% praziquantel.

The anthelmintic paste described in US2010/0286154 A1 is a water-based suspension of praziquantel in the form of a paste comprising cyclodextrin, which is devoid of organic solvents, designed to provide for a suitable palatability for an oral application to warm-blooded animals, in particular to cats and dogs. Such cyclodextrin complexation would provide for the enhanced solubilisation of hydrophobic drugs. However, cyclodextrin complexed drugs do show a significantly changed behaviour and cyclodextrin would have a pharmacological effect itself (Stella et al. 2008. Toxicologic Pathology 36:30-42).

US2005/0106202 A1 describes an endoparaciticidal gel composition that allows for higher concentrations of praziquantel (10-15% w/w) and bioavailability over a prolonged period of time. The gel comprises the active ingredients in a clear non-acqueous solution.

NWC tend to spit when they are unwilling or even unable to swallow the enormous quantities necessary when using conventional drugs. The tendency of spitting is also apparent when llamas and alpacas do not like the taste of a medicinal product. Hence there is an evident risk of inadequate and incomplete dose application. Inadequate treatment leads to further progression of the pathological changes in diseased animals and underdosing of the drug is greatly associated with the risk of emergence of resistance.

Therefore, it is the object of the present invention to provide for an alternative formulation of praziquantel, which is accepted by animals even at high doses, in particular by camelids. There is the further object to provide for a palatable formulation comprising therapeutic agents for use in the treatment of camelids.

### Summary of the invention

The object is solved by the subject matter as claimed.

According to the invention there is provided an oral hydrosuspension gel comprising at least 15% (w/w) free praziquantel, at least 30% water and at least one gelifyer. Such gel comprises the praziquantel in the uncomplexed form, and is devoid of any inclusion complex, such as obtained with cyclodextrins. Therefore, the gel according to the invention is devoid of a cyclodextrin inclusion complex. Such an aqueous gel is also called suspension gel or suspension hydrogel.

The gel according to the invention turned out to be highly accepted by large animals, in particular camelids, and provides the basis for a palatable gel of high praziquantel concentration for use in the treatment of camelids.

The gel specifically comprises 20-50% (w/w) praziquantel, preferably 22-40%, most preferred about (+/- 1%) 25%.

The concentration of compounds in the gel according to the invention typically is provided as a percentage (%) number, which is understood as weight percentage (w/w) .

Specifically, the gel according to the invention comprises the gelifyer that is typically a gelling agent, such as a hydrocolloid. Specifically the gelifyer is selected from the group consisting of a cellulose derivative, such as water soluble cellulose ether, including hydroxyethyl cellulose, methyl hydroxyethyl cellulose, hydroxy ethyl methyl cellulose, hydroxyl propyl cellulose and hydroxyl propylmethylcellulose; starches, such as hydroxyethyl starch; natural gums, such as xanthum gum; and synthetic polymers, such as polymers and copolymers of polyvinylpyrrolidone, and carbomeres, including also mixtures thereof.

Preferably a cellulose derivative is employed, such as hydroxylethyl cellulose. Specifically, Natrosol® may be used, which is a hydroxyethylcellulose polymer and a hydroxyethylether of cellulose. Natrosol® is commercially available (e.g. of Ashland Inc., Covington, U.S.A.) in different degree of polymerization and viscosity types, a preferred gelifyer has a low degree of polymerization such as less than 5000, preferably less than 3000, and a low viscosity at room temperature, such as Natrosol® 250 G or L. The amount and the type of the gelifyer in the formulation may be selected to provide the desired product consistency and/or viscosity to facilitate oral administration, in particular for warm-blooded animals.

Specifically, the gel according to the invention comprises at least one gelifying excipient. The gelifying excipients as used according to the invention specifically are understood as plasticizers typically used to moisture or humidify the gelifyer as used according to the invention, so to obtain a homogenous mixture or ease the gelling process. As preferred gelifying excipients water miscible polar organic solvents may be used.

According to specifically preferred embodiments, the gel according to the invention comprises the gelifyer at a concentration of 0,1-10% and optionally a gelifying excipient at a concentration of 10-30%.

Specifically, the gelifying excipient is selected from the group consisting of 1,2-propane diol and glycerol. Such gelifying excipient is particularly preferred with a gelifyer selected from the group consisting of cellulose derivatives, such as hydroxyethyl cellulose. The preferred gelifying excipient is sweet-tasting and gel-forming, such as 1,2-propane diol, which has the additional advantage of having an antimicrobial and eventually preserving effect at the concentrations employed.

The preferred gelifyer provides for a suitable viscosity of the suspension gel. Specifically, the viscosity of the gel according to the invention is ranging from 1,000 to 500,000 cp at 20°C, preferably 5,000 to 100,000, more preferably 8,000 to 80,000 cp.

The preferred water content of the gel according to the invention is at least 40%, more preferred at least 50%, even more preferred at least 60%. Such gels are specifically preferred comprising at least 50% water and having a viscosity ranging from 1,000 to 500,000 cp at 20°C, or any of the preferred viscosity ranges.

According to a specific embodiment, the gel comprises praziquantel as the sole therapeutic agent.

According to another specific embodiment, the gel comprises a further additional therapeutic agent selected from the group consisting of antibiotics, chemotherapeutics, antiparasitic, antimycoplasmic, anthelmintic, antiprotozoic agents, antimycotics, vitamins, nutrients, macrominerals, trace minerals, analgetics, sedative agents, anti-inflammatory agents, anti-ulcera agents, and agents for treating neurological, respiratory, urinary, gastro-intestinal, hepatic or metabolic disorders, or combinations thereof.

Preferred combinations are e.g. agents from the group of antibiotics in combination with antiparasitics; nutrients-vitamines-macrominerals-trace minerals in any combination of two or more; or anti-inflammatory in combination with anti-ulcera agents. Further preferred combinations are agents combined with other agents of the same group, e.g. praziquantel in combination with another anthelmintic agent.

Specifically, the gel according to the invention is presented as a medicament in a ready-to-use application form, preferably together with an integrated dosing device. Preferably, the device is a metered dosing device, such as a calibrated syringe, to enable the oral administration of specific amounts of the therapeutic agents.

Specifically, the gel according to the invention provides for the use in preventing and/or treating infection by endoparasites, such as Dicrocoeliosis, in particular providing a praziquantel dose ranging from 10 to 100 mg/kg, preferably 20 to 80 mg/kg, more preferred 40 to 60 mg/kg, particularly for treating camelids, such as NWC.

According to a specific embodiment the gel is administered once to several times a year, preferably 1 to 6 times a year.

Therefore, further disclosed is a method of treating subjects for the prophylaxis and/or treatment of disease conditions resulting from endoparasitic infection, specifically subjects at risk of or suffering from infection by endoparasites, such as trematodes, nematodes, cestodes and protozoan parasites, in particular for treating camelids, such as NWC, by administering an effective amount of the gel according to the invention.

The oral hydrosuspension gel formulation according to the invention is even more broadly applicable for treating camelids. Therefore, according to a further aspect, the invention refers to a gel for use in the oral treatment of camelids, which is based on a hydrosuspension gel, further comprising
- 1-50% therapeutic agent that is sparingly soluble to insoluble in water,
- 0.1-10% gelifyer selected from the group consisting of cellulose derivatives,
- 10-30% gelifying excipient selected from the group consisting of 1,2-propane diol and glycerol, wherein said gelifyer is at a concentration of 0.1 - 10%.

Such gel may comprise the hydrosuspension gel as a sole component, e.g. essentially consisting of such gel, or else as a multicomponent composition or set comprising further components.

Specifically, a method of treating camelids is disclosed to prevent or treat various disease conditions by oral administration of a suspension gel according to the invention, which is characterized by the high concentration of 1,2-propane diol or glycerol, which turned out to be well-accepted by the camelids.

The therapeutic agent may be selected from the group consisting of antibiotics, chemotherapeutics, antiparasitic, antimycoplasmic, anthelmintic, antiprotozoic agents, antimycotics, vitamins, nutrients, macrominerals, trace minerals, analgetics, sedative agents, anti-inflammatory agents, anti-ulcera agents, and agents for treating neurological, respiratory, urinary, gastro-intestinal, hepatic or metabolic disorders, or combinations thereof.

Preferred active ingredients or combinations are e.g. praziquantel as a sole therapeutic agent or in combination with an additional therapeutic agent of the same group of therapeutic agents or of a different group.

Preferred combinations are e.g. agents from the group of antibiotics in combination with antiparasitics; nutrients-vitamines-macrominerals-trace minerals in any combination of two or more; or anti-inflammatory in combination with anti-ulcera agents. Further preferred combinations are agents combined with other agents of the same group, e.g. praziquantel in combination with another anthelmintic agent.

Specifically, the gel according to the invention is characterized by the therapeutic agent, such as praziquantel and/or other targets that are sparingly soluble, which is comprised in the suspension in the undissolved form, e.g. with particle sizes of 0.5 - 300 µm, specifically up to 200 µm. The therapeutic agent is provided in the free form, i.e. in the uncomplexed form, e.g. in a formulation devoid of cyclodextrins and cyclodextrin inclusion complexes. Thus, the therapeutic agent is dispersed in the aqueous gel as a free active principle.

Specifically, the cellulose derivative is a water soluble cellulose ether, including hydroxyethyl cellulose, methyl hydroxyethyl cellulose, hydroxy ethyl methyl cellulose, hydroxyl propyl cellulose and hydroxyl propylmethylcellulose. The preferred gelifyer provides for a suitable viscosity of the suspension gel.

Specifically, preferred gels according to the invention are colourless, e.g. white or milky hydrosuspension, or may be colored as well.

Further preferred physicochemical characteristics of the medicament according to the invention, e.g. viscosity, pH, etc, are characterised by the specific embodiments of the hydrosuspension gel as detailed above.

### Detailed description of the invention

Specific terms as used throughout the specification have the following meaning.

The term "effective amount" of a therapeutic agent or ingredient, as used herein shall mean an amount of the agent sufficient enough to have a net positive effect upon administration. A therapeutically effective amount of the therapeutic agent or ingredient, is the amount as needed for effective prophylaxis and/or therapy, and particularly will cause the prevention or substantial relief of symptoms, e.g. when administered repeatedly over time. Effective amounts of the therapeutic agent or ingredient, will vary with the particular condition or conditions being treated, the severity of the condition, the duration of the treatment, the specific components of the composition being used, and like factors.

An effective amount of praziquantel specifically provides for the efficacy of treatment as determined by calculation of the extensity effect (percentage reduction in the number of animals excreting eggs two weeks after treatment). For example, the effective amount provides for the extensity effect of at least 50%, preferably at least 60%, 70%, 80% or 90%, meaning that the majority of animals remains unaffected upon administration, so that no eggs are determined in the faeces after two weeks.

Praziquantel (CAS number 55268-74-1) is understood herein as a synthetic, pyrazinoisoquinoline derivative anthelmintic agent, IUPAC Name: (RS)-2-Cyclohexylcarbonyl- 2,3,4,6,7,11b-hexahydro-1H-pyrazino(2,1-a) isoquinolin- 4-on, USP: 2-(cyclohexylcarbonyl)-1,2,3,6,7,11b - hexahydro-4H-pyrazino(2,1-a) isoquinolin-4-one; molecular formula; C₁₉H₂₄N₂O₂, in the form of the racemate or one of its isomers. Praziquantel is a white to nearly white crystalline powder of bitter taste. The compound is stable under normal conditions and melts at 136-140°C with decomposition. Praziquantel is easily soluble in chloroform and dimethylsulfoxide, soluble in ethanol and hardly soluble in water (up to 400 mg/L, sparingly soluble).

The term "water solubility" as used herein refers to the amount of a substance (e.g. a solid) that will dissolve in water. Solubility is generally determined at temperatures between 15°C and 25°C and expressed as w/v. As used herein, a sparingly soluble solute in liquid is understood as 1 in 30 to 1 in 10,000, and a practically insoluble or insoluble one: 1 in more than 10,000. Solubility ranges are available in published pharmacopoeias, including United States Pharmacopoeia (USP) and European Pharmacopoeia (EP).

The term "gelifyer" as used herein refers to gelling agents, including thickening agents that provide a suitable viscosity to a liquid and are in an amount that is sufficient to hold the active agent which is sparingly soluble to insoluble in water in a suspension. The thickening agent can be substantially chemically inert. The thickening agent can be synthetic or naturally occurring, e.g. of vegetable origin, for example celluloses and derivatives of methyl cellulose, hydroxyethyl cellulose, hydroxypropyl cellulose, hydroxypropyl methyl cellulose and carboxymethyl cellulose, starches and dextrins.

The term "pharmaceutically acceptable" as used herein refers to a material which is tolerable and not biologically or otherwise undesirable, i.e. the material may be administered to an individual along with the selected active agent without causing any undesirable biological effects.

The term "suspension gel" as used herein refers to a colloidal or particulate suspension or dispersion of a solid dispersed in a liquid, provided as a gel, which is an apparently solid or semi-solid, jelly-like material. Specific gels according to the invention display thixotropy, becoming fluid when agitated, but resolidify when resting. A hydrosuspension gel, such as a hydrosuspension gel according to the invention, is based on an aqueous suspension, comprising solids dispersed in an aqueous medium, such as containing at least 30%, specifically at least 40%, at least 50% or at least 70% water (w/w).

The suspension gel according to the invention specifically forms a matrix which should be as inert as possible with respect to the therapeutic agent and its bioavailability, thus specifically avoiding inclusion complexes, such as cyclodextran complexes. The desired rheological properties may be obtained by means of a gelifyer, optionally in combination with suitable flow modifiers which impart the properties to the gel and maintain them as a function of time.

The term "therapeutic agent" or "active agent" as used herein refers to a pharmaceutically active agent having a pharmacological, prophylactic or therapeutic effect in animals, including warm-blooded animals, specifically camelids, but also other species, including human beings.

Therefore, the invention provides for a galenic formulation of a therapeutic agent, such as praziquantel, for oral use, specifically adequate for NWC treatment. The oral suspension gel according to the invention may cover several essential requirements for pharmacological treatment of NWC:
i) it allows higher concentrations of the drug than the formulations known so far,
ii) the taste of the formulation is highly palatable to NWC, so that the applied dose is willingly accepted and swallowed by the animals without the attempt of spitting,
iii) the application volume is small enough to guarantee an easy and safe therapy with the complete dose required for a single oral treatment in NWC,
iv) emergence of resistance of the parasite due to partial loss of the drug caused by spitting or other difficulties when applying huge volumes orally to NWC, is avoided.

The gel according to the invention may additionally contain one or more agents elected from the group of sweetening agents, flavouring agents, colouring agents, antioxidants and preserving agents in order to produce pharmaceutically elegant and palatable preparations, which may or may not comprise further pharmaceutically acceptable carriers.

Suitable sweeteners include sucrose, lactose, glucose, maltodextrin, aspartame, sodium cyclamate, acesulfame, saccharin, stevia, neohesperidin-dihydrochalcone, glycyrrhizinic acid, derivatives thereof, and mixtures thereof; these sweetening agents are particularly used to mitigate the bitter tastes and harsh tastes of praziquantel or other therapeutic agents. When 1,2-propane diol or glycerol is used as gelifying excipient, the suspension gel specifically would already have a sweet taste because of the nature and concentration of the gelifying excipient. Therefore, further sweetening agents may be avoided in the presence of at least 10% 1,2-propane diol or glycerol.

Suitable flavouring agents include maltol, glycin, succinic acid, derivatives thereof, and mixtures thereof Suitable preservatives include sodium benzoate, sorbic acid, vitamin E, alpha-tocopherol, gamma-tocopherol, tocopherols and tocotrienols, ascorbic acid, benzyl alcohol or combinations thereof. Suitable antioxidants may be tocopherols and tocotrienols. The suspension gel can further contain alcohol or be alcohol-free.

Other pharmaceutically acceptable excipients known in the art may be used, specifically those that are licensed for veterinary purposes, with the proviso, that the further excipients in question would not significantly affect the rheological properties of the matrix gel.

The gel according to the invention is devoid of cyclodextrin or cyclodextrin inclusion complexes that would increase the apparent water solubility of the therapeutic agent.

The gel according to the invention is preferably devoid of oils, since oily gels would not easily be accepted by the animals.

Typically, the gelifyer and optionally the gelifying excipient are added to an aqueous medium containing one or more therapeutic agents in a suspension and optionally further excipients, which is then stirred to form a hydrosuspension gel.

According to a specific aspect, the gel according to the invention is manufactured by a standardised process, comprising
- providing a therapeutic agent,
- mixing the therapeutic agent with the gelifyer in an appropriate amount,
- optionally adding a gelifying excipient to the mixture,
- dispersing the mixture in an appropriate amount of water, and
- stirring until the visous gel is obtained.

The suspension may be uniformly stirred and homogenized optionally with heating using a mechanical stirrer equipped with an appropriate mechanism for heating and cooling; the resulting homogeneous suspension may then be filled and cooled to solidify in an optionally chosen container, whereby the suspension gel preparation of the present invention can be prepared.

Preferably, the gel is manufactured during a short period of time. Although the gelling time varies depending on the amount and type of the gelifyer used, the suspension usually gels to yield a suspension gel when allowed to mix or stand at normal temperature, e.g. for 1 minute to 5 hours. For example, mixing the ingredients and stirring may be done within 1 minute to five hours, preferably less than 2 hours.

The particle sizes of the therapeutic agent typically have an average diameter of in the range of about 0.5-300 µm, usually up to 200 µm, specifically within the range of 1-100 µm, more specifically 5 - 20 µm, as measured using techniques known in the art, including, for example, visual inspection using a microscope.

Specifically, the formulation may be provided as aseptic product.

The preferred formulation is storage stable at room temperature, e.g. at least 6 months, 1 year or 2 years, such as to provide a storage stable product, eventually in a final package.

The preferred oral drug delivery system or dispenser used in accordance to the invention may allow for the application of a preferred lick gel, e.g. with a syringe to place the gel onto an animal's tongue, or together with a dosing spoon or other metered device, dispenser, including pumps, or any other manual or automatic application device.

For example, the gel according to the invention may be prepared for dispensing by packing the gel in a dispenser pack having a metering pump for dispensing metered amounts of the gel.

A dispenser is preferably used together with the gel according to the invention, which is equipped with an element for metering the effective amount of the therapeutic agents. For example, the dispenser may have the ability to deliver a therapeutic dose of low volume by one or two depressions of the dispenser which, in some cases, requires a high concentration of therapeutic agent in the suspension gel.

The gel may specifically be contained in a device having an internal volume of 1 to 100 ml and which is preferably provided with a metering compartment, e.g. not exceeding 1-50 ml in volume corresponding to the unit dose to be dispensed, e.g. to dispense a therapeutic dose in one or two depressions per therapeutic dose and in that the contents of the dispenser are sufficient for at least 1 - 5 days' treatment.

Dispensers of the type in question are widely used in the veterinary field. For example, they may be formed as a syringe or else by a cylindrical body of plastic, aluminium or glass filled with product which enables discharging the product in an appropriate way.

The suspension gel according to the invention may have an appropriately balanced viscosity, that is sufficiently viscous to hold the therapeutic agent in a suspension, but not so viscous so as to be unable to be expelled from a container, such as a syringe, or other dispenser. The suspension gel specifically may have a viscosity that achieves a pourable gel and that allows the gel to be expelled easily and evenly from a container.

Specifically, the amount and type of gelifyer and optional gelifying excipient is used to form a jelly-like semisolid formulation, e.g. a pseudoplastic and more or less thixotropic water-dispersible gel. The required pseudoplasticity corresponds to a resistance of the gel which is plastic up to a certain shear limit, but breaks beyond that limit. Thixotropy is understood to be the property which the gel has of becoming less viscous when subjected to constant shearing (constant friction in the metering element during dispensing) and returning to its initial structure after removal of the shearforce and standing for a sufficient time. The consistency of the gel should be such that it can be discharged from a syringe or pumped, is sufficiently deformable for exactly filling the volume of the metering compartment without becoming stringy and can be discharged from the compartment and broken to form a dose without running during dispensing. In addition, this property of pseudoplasicity enables the gel to be deposited onto a support, for example a spoon, and to adhere sufficiently to the support without dropping, even when the spoon is turned upside down. In addition, because the gel is dispersible in water, it does not adhere to the mucosa, but at the same time is not destructured in the mouth so that it is easy to swallow.

The preferred devices may provide for the gel in a single dosage form, which is particularly used to treat a single or a small number of animals. Herds would preferably be treated with multiple dosage forms.

Dosage unit forms will generally contain between 5 to 10,000 mg of a therapeutic agent depending on the host treated and the potency of the therapeutic agent.

Thus, the pharmaceutical composition according to the invention is characterized in that the therapeutic agent is homogeneously distributed in a pseudoplastic hydrosuspension or water-dispersible gel which does not run during dispensing and which is organoleptically acceptable.

The therapeutic agent preferably is praziquantel as a sole therapeutic agent or in a combination of other therapeutic agents.

Alternative or other therapeutic agents are specifically selected from the group of therapeutic agents listed above, in particular the agents mentioned below (see also The Merck Veterinary Manual, 10th Edition, Cynthia M. Kahn (Editor), Scott Line (Associate Editor), John Wiley & Sons, 2010; "Medicine and Surgery of Camelids" Murray E. Fowler, John Wiley & Sons, 2010).

Exemplified antibiotics and chemotherapeutics include, for example, penicillins, cephalosporins, macrolides, quinolones, sulfonamides, aminoglycosides and tetracyclines.

Exemplified antiparasitics include, for example, benzimidazoles, macrocyclic lactones, imidazothiazoles and tetrahydropyrimidines.

Exemplified antimycoplasmics include, for example, tylosin.

Exemplified anthelmintic include, for example, praziquantel, and further benzimidazoles, macrocyclic lactones, imidazothiazoles and tetrahydropyrimidines.

Exemplified antiprotozoic agents include, for example, sulphonamides, ionophore antibiotics and triazone compounds.

Exemplified antimycotics include, for example, azole derivatives, polyene antibiotics and antifungal antibiotics.

Exemplified vitamins include, for example, vitamin A, vitamin B 1-12, vitamin D, and vitamin E.

Exemplified nutrients include, for example, chondroitin sulphate, menbutone, flavonoids, antacids and proprionate.

Exemplified macrominerals include, for example, calcium, magnesium, potassium, iron, sodium and chloride.

Exemplified trace minerals for example selenium, zinc and copper.

Exemplified analgetics include, for example, steroidale and non steroidale analgetics.

Exemplified sedative agents include, for example, acepromazine and benzodiazepines.

Exemplified anti-inflammatory agents include, for example, non steroidal anti-inflammatory drugs.

Exemplified anti-ulcera agents include, for example, antacids, omeprazole, misoprostol, sucralfate and H2 receptor antagonists.

Exemplified agents for treating neurological disorders include, for example, opioid analgesics and benzimidazoles.

Exemplified agents for treating respiratory disorders include, for example, antitussives, bronchodilators, mucolytics and respiratory stimulants.

Exemplified agents for treating urinary disorders include, for example, acidifiers and alkalinisers.

Exemplified agents for treating gastro-intestinal disorders include, for example, adsorbents, antidiarrhoeal drus, antispasmodics, erythromycin, paraffins and silicones.

Exemplified agents for treating hepatic disorders include, for example, silymarin, glucose precursors and menbutone.

Exemplified agents for treating metabolic disorders include, for example, glucose precursors.

An exemplary gel acceptance test shows the ease of oral administration to the animals, even with therapeutic agents, such as praziquantel, which are bad-tasting per se. Therefore, small amounts of the gel are placed into the oral cavity or onto the lips of the animal, and the behaviour of the animal is watched. A palatable gel according to the invention is typically licked by the animals without any signs of resistance.

For example, the efficacy of the suspension gel according to the invention comprising a high concentration of praziquantel, e.g. 25%, prepared using hydroxylethyl cellulose as gelifyer and 1,2-propane diol as gelifying excipient in an aqueous suspension, was tested in two different dose rates and found to be well accepted.

The foregoing description will be more fully understood with reference to the following examples. Such examples are, however, merely representative of methods of practicing one or more embodiments of the present invention and should not be read as limiting the scope of invention.

### Examples

### Example 1: Preparation of an exemplary suspension gel

An exemplary suspension gel was prepared as follows:
1. Weighing 25 g praziquantel (USP 30, Richter Pharma GmbH, Germany) and 4 g hydroxyethylcellulose 250 G Pharm (Ph. Eur. 7.0, e.g. FAGRON GmbH & Co. KG, Germany) in a paten of suitable size
2. Mixing to a homogeneous mixture (to reduce the formation of lumps)
3. Adding 20 g propylene glycol
4. Short mixing (10 - 20 seconds) at room temperature to moisture the solid ingredients
5. Adding the total amount of water: 51 g (resulting in 100 g oral gel)
6. Stirring for one minute. The gel is then ready and has its final consistency: viscosity = 25000 cp measured with a Rotational viscometer (Haake RT 20) at 20 rotations per minute (21 °C)
7. As experience shows continued stirring and resting provides for a better result (reduced formation of lumbs)
8. Transferring into the application tools (e.g. syringes)

### Example 2: Acceptance test with llamas

34 llamas naturally infected with *Dicrocoelium dentriticum* were randomly assigned to one of two study groups. Group 1 was treated with a single oral application of the gel prepared according to Example 1 (concentration 25%) of 25 mg/kg BW (body weight) and animals of group 2 with a single oral application of 50 mg/kg BW. The animals accepted the administered gel well. Coprological examinations were performed before and two weeks after treatment. The efficacy was determined by calculation of the extensity effect (percentage reduction in the number of animals excreting eggs two weeks after treatment). The efficacy of the invented formulation in the llamas studied was 88.2% when treated at a dose rate of 50 mg/kg BW. Therapy with 25 mg/kg BW showed an efficacy of 82.3%.

## Claims

1. Oral hydrosuspension gel comprising at least 15% (w/w) free praziquantel, at least 30% water and at least one gelifyer, which is devoid of cyclodextrins and cyclodextrin inclusion complex.

2. Gel according to claim 1, comprising 20-50% (w/w) praziquantel.

3. Gel according to claims 1 or 2, wherein the gelifyer is selected from the group consisting of a cellulose derivative, such as water soluble cellulose ether, including hydroxyethyl cellulose, methyl hydroxyethyl cellulose, hydroxy ethyl methyl cellulose, hydroxyl propyl cellulose and hydroxyl propylmethylcellulose; starches, such as hydroxyethyl starch; natural gums, such as xanthum gum; and synthetic polymers, such as polymers and copolymers of polyvinylpyrrolidone, and carbomeres.

4. Gel according to any of claims 1 to 3, which further comprises at least one gelifying excipient.

5. Gel according to any of claims 1 to 4, comprising the gelifyer at a concentration of 0.1-10% and optionally a gelifying excipient at a concentration of 10-30%.

6. Gel according to claim 4 or 5, wherein the gelifying excipient is selected from the group consisting of 1,2-propane diol and glycerol.

7. Gel according to claim 6, wherein the gelifyer is selected from the group consisting of cellulose derivatives.

8. Gel according to any of claims 1 to 7, which has a viscosity ranging from 1,000 to 500,000 cp at 20°C.

9. Gel according to any of claims 1 to 8, comprising a further therapeutic agent selected from the group consisting of antibiotics, chemotherapeutics, antiparasitic, antimycoplasmic, anthelmintic, antiprotozoic agents, antimycotics, vitamins, nutrients, macrominerals, trace minerals, analgetics, sedative agents, anti-inflammatory agents, anti-ulcera agents, and agents for treating neurological, respiratory, urinary, gastro-intestinal, hepatic or metabolic disorders, or combinations thereof.

10. Gel according to any of claims 1 to 9, presented in a metered dosing device for oral administration.

11. Gel according to any of claims 1 to 10, for use in preventing and/or treating infection by endoparasites, in particular providing a praziquantel dose ranging from 10 to 100 mg/kg.

12. Gel according to claim 1 for use in the oral treatment of camelids, comprising at least 15% (w/w) free praziquantel, at least 30% water, at least one gelifyer selected from the group consisting of cellulose derivatives, which is devoid of cyclodextrins and cyclodextrin inclusion complex, further comprising
- 1-50% therapeutic agent that is sparingly soluble to insoluble in water,
- 0.1-10% gelifyer,
- 10-30% gelifying excipient selected from the group consisting of 1,2-propane diol and glycerol,
wherein said gelifyer is at a concentration of 0.1 - 10%.

13. Gel according to claim 12 for use in the oral treatment of camelids, wherein the therapeutic agent is selected from the group consisting of antibiotics, chemotherapeutics, antiparasitic, antimycoplasmic, anthelmintic, antiprotozoic agents, antimycotics, vitamins, nutrients, macrominerals, trace minerals, analgetics, sedative agents, anti-inflammatory agents, anti-ulcera agents, and agents for treating neurological, respiratory, urinary, gastro-intestinal, hepatic or metabolic disorders, or combinations thereof.

14. Gel according to any of claims 12 or 13 for use in the oral treatment of camelids, wherein the gelifyer is a water soluble cellulose ether, such as selected from the group consisting of hydroxyethyl cellulose, methyl hydroxyethyl cellulose, hydroxy ethyl methyl cellulose, hydroxyl propyl cellulose and hydroxyl propylmethylcellulose.

## Patentansprüche

1. Orales Hydrosuspensionsgel, umfassend zumindest 15 % (w/w) freies Praziquantel, zumindest 30 % Wasser und zumindest ein Geliermittel, das frei von Cyclodextrinen und Cyclodextrin-Einschluss-Komplexen ist.

2. Gel nach Anspruch 1, umfassend 20-50 % (w/w) Praziquantel.

3. Gel nach den Ansprüchen 1 oder 2, wobei das Geliermittel aus der Gruppe ausgewählt ist, die aus einem Cellulose-Derivat, wie etwa wasserlöslichem Celluloseether, der Hydroxyethylcellulose, Methylhydroxyethylcellulose, Hydroxyethylmethylcellulose, Hydroxylpropylcellulose und Hydroxylpropylmethylcellulose beinhaltet; Stärken, wie etwa Hydroxyethylstärke; natürlichen Gummis, wie etwa Xanthangummi; und synthetischen Polymeren, wie etwa Polymeren und Copolymeren von Polyvinylpyrrolidon, und Carbomeren besteht.

4. Gel nach einem der Ansprüche 1 bis 3, das ferner zumindest ein Gelierhilfsmittel umfasst.

5. Gel nach einem der Ansprüche 1 bis 4, welches das Geliermittel in einer Konzentration von 0,1-10 % und gegebenenfalls ein Gelierhilfsmittel in einer Konzentration von 10-30 % umfasst.

6. Gel nach Anspruch 4 oder 5, wobei das Gelierhilfsmittel aus der Gruppe ausgewählt ist, die aus 1,2-Propandiol und Glycerin besteht.

7. Gel nach Anspruch 6, wobei das Geliermittel aus der Gruppe ausgewählt ist, die aus Cellulosederivaten besteht.

8. Gel nach einem der Ansprüche 1 bis 7, das eine Viskosität aufweist, die von 1.000 bis 500.000 cp bei 20 °C reicht.

9. Gel nach einem der Ansprüche 1 bis 8, das ein weiteres therapeutisches Mittel umfasst, das aus der Gruppe ausgewählt ist, die aus Antibiotika, Chemotherapeutika, Antiparasitika, antimykoplasmatischen Mitteln, Anthelminthika, antiprotozoischen Mitteln, Antimykotika, Vitaminen, Nährstoffen, Makromineralien, Spurenelementen, Analgetika, Beruhigungsmitteln, entzündungshemmenden Mitteln, Mitteln gegen Geschwüre und Mitteln zur Behandlung von neurologischen, respiratorischen, Harnwegs-, Magen-Darm-, Leber- oder Stoffwechselstörungen oder Kombinationen davon besteht.

10. Gel nach einem der Ansprüche 1 bis 9, das in einer Mess-Dosiervorrichtung zur oralen Verabreichung dargereicht wird.

11. Gel nach einem der Ansprüche 1 bis 10, zur Verwendung bei der Vorbeugung und/oder Behandlung einer Infektion durch Endoparasiten, wobei das Gel insbesondere eine Praziquantel-Dosis bereitstellt, die von 10 bis 100 mg/kg reicht.

12. Gel nach Anspruch 1 zur Verwendung bei der oralen Behandlung von Cameliden, umfassend zumindest 15 % (w/w) freies Praziquantel, zumindest 30 % Wasser und zumindest ein Geliermittel, das aus der Gruppe ausgewählt ist, die aus Cellulose-Derivaten besteht, und das frei von Cyclodextrinen und Cyclodextrin-Einschluss-Komplexen ist, ferner umfassend
- 1-50 % eines therapeutischen Mittels, das in Wasser gering löslich bis unlöslich ist,
- 0,1-10 % Geliermittel,
- 10-30 % Gelierhilfsmittel, das aus der Gruppe ausgewählt ist, die aus 1,2-Propandiol und Glycerin besteht,
wobei das Geliermittel in einer Konzentration von 0,1 - 10 % vorliegt.

13. Gel nach Anspruch 12 zur Verwendung bei der oralen Behandlung von Cameliden, wobei das therapeutische Mittel aus der Gruppe ausgewählt ist, die aus Antibiotika, Chemotherapeutika, Antiparasitika, antimykoplasmatischen Mitteln, Anthelminthika, antiprotozoischen Mitteln, Antimykotika, Vitaminen, Nährstoffen, Makromineralien, Spurenelementen, Analgetika, Beruhigungsmitteln, entzündungshemmenden Mitteln, Mitteln gegen Geschwüre und Mitteln zur Behandlung von neurologischen, respiratorischen, Harnwegs-, Magen-Darm-, Leber- oder Stoffwechselstörungen oder Kombinationen davon besteht.

14. Gel nach einem der Ansprüche 12 oder 13 zur Verwendung bei der oralen Behandlung von Cameliden, wobei das Geliermittel ein wasserlöslicher Celluloseether ist, etwa ausgewählt aus der Gruppe, die aus Hydroxyethylcellulose, Methylhydroxyethylcellulose, Hydroxyethylmethylcellulose, Hydroxylpropylcellulose und Hydroxylpropylmethylcellulose besteht.

## Revendications

1. Gel oral en suspension aqueuse comprenant au moins 15 % (p/p) de praziquantel libre, au moins 30 % d'eau et au moins un gélifiant qui est exempt de cyclodextrines et de complexe d'inclusion de cyclodextrines.

2. Gel selon la revendication 1, comprenant de 20 à 50 % (p/p) de praziquantel.

3. Gel selon les revendications 1 ou 2, dans lequel le gélifiant est sélectionné dans le groupe constitué d'un dérivé de cellulose, comme un éther de cellulose soluble dans l'eau, y compris l'hydroxyéthylcellulose, la méthyl hydroxyéthyl cellulose, l'hydroxyéthyl méthyl cellulose, l'hydroxypropyl cellulose et l'hydroxypropylméthylcellulose ; des amidons, comme l'amidon hydroxyéthylé ; des gommes naturelles, comme la gomme de xanthane ; et des polymères synthétiques, comme des polymères et copolymères de polyvinylpyrrolidone et des carbomères.

4. Gel selon l'une quelconque des revendications 1 à 3, qui comprend en outre au moins un excipient gélifiant.

5. Gel selon l'une quelconque des revendications 1 à 4, comprenant le gélifiant à une concentration de 0,1 à 10 % et en option, un excipient gélifiant à une concentration de 10 à 30 %.

6. Gel selon la revendication 4 ou 5, dans lequel l'excipient gélifiant est sélectionné dans le groupe constitué de propane-1,2 diol et de glycérine.

7. Gel selon la revendication 6, dans lequel le gélifiant est sélectionné dans le groupe constitué de dérivés de cellulose.

8. Gel selon l'une quelconque des revendications 1 à 7, qui a une viscosité s'étendant de 1000 à 500 000 cP à 20 °C.

9. Gel selon l'une quelconque des revendications 1 à 8, comprenant un agent thérapeutique supplémentaire sélectionné dans le groupe constitué d'antibiotiques, d'agents chimiothérapeutiques, d'antiparasitaires, d'anti-mycoplasmiques, d'anthelminthiques, d'antiprotozoaires, d'antifongiques, de vitamines, de nutriments, de macrominéraux, d'oligo-éléments, d'analgésiques, d'agents sédatifs, d'agents anti-inflammatoires, d'agents anti-ulcéreux, et d'agents pour le traitement de troubles neurologiques, respiratoires, urinaires, gastro-intestinaux, hépatiques ou métaboliques, ou des combinaisons de ceux-ci.

10. Gel selon l'une quelconque des revendications 1 à 9, présenté dans un dispositif doseur pour l'administration orale.

11. Gel selon l'une quelconque des revendications 1 à 10, pour utilisation dans la prévention et/ou le traitement d'une infection par des endoparasites, en particulier fournissant une dose de praziquantel s'étendant de 10 à 100 mg/kg.

12. Gel selon la revendication 1 pour utilisation dans le traitement oral des camélidés, comprenant au moins 15 % (p/p) de praziquantel libre, au moins 30 % d'eau, au moins un gélifiant sélectionnant dans le groupe constitué de dérivés de cellulose, qui est exempt de cyclodextrines et de complexe d'inclusion de cyclodextrines, comprenant en outre
- 1 à 50 % d'un agent thérapeutique qui est faiblement soluble à insoluble dans l'eau,
- 0,1 à 10 % d'un gélifiant,
- 10 à 30 % d'un excipient gélifiant sélectionné dans le groupe constitué de propane-1,2 diol et de glycérine,
dans lequel ledit gélifiant est à une concentration de 0,1 à 10 %.

13. Gel selon la revendication 12 pour utilisation dans le traitement oral des camélidés, dans lequel l'agent thérapeutique est sélectionné dans le groupe constitué d'antibiotiques, d'agents chimiothérapeutiques, d'antiparasitaires, d'anti-mycoplasmiques, d'anthelminthiques, d'antiprotozoaires, d'antifongiques, de vitamines, de nutriments, de macrominéraux, d'oligo-éléments, d'analgésiques, d'agents sédatifs, d'agents anti-inflammatoires, d'agents anti-ulcéreux, et d'agents pour le traitement de troubles neurologiques, respiratoires, urinaires, gastro-intestinaux, hépatiques ou métaboliques, ou des combinaisons de ceux-ci.

14. Gel selon l'une quelconque des revendications 12 ou 13, pour utilisation dans le traitement oral des camélidés, dans lequel le gélifiant est un éther de cellulose soluble dans l'eau, comme ceux sélectionnés dans le groupe constitué d'hydroxyéthylcellulose, de méthyl hydroxyéthyl cellulose, d'hydroxyéthyl méthyl cellulose, d'hydroxypropyl cellulose et d'hydroxypropylméthylcellulose.
